# EUROPEAN PATENT APPLICATION

(11) **EP 1 215 214 A1**
(43) Date of publication of application: **19.06.2002**
(21) Application number: 01310137.3
(22) Date of filing: 04.12.2001
(51) Int. Cl.: C07K 14/705, C12N 15/12, C07K 16/28, G01N 33/68, C12N 5/10, A61K 39/395, A61K 38/17, C12Q 1/68

(54) **Novel polypeptide**

(30) Priority: 18.12.2000 GB 0030855; 17.01.2001 GB 0101222
(71) Applicant: Pfizer Limited, Sandwich, Kent CT13 9NJ (GB); PFIZER INC., New York, N.Y. 10017 (US)
(72) Inventor: Fidock, Mark David, Sandwich, Kent, CT13 9NJ (GB)
(74) Representative: Hayles, James Richard

(57) **Abstract**

Polynucleotide and polypeptide sequences are described. The polypeptide sequences comprise one or more of: (a) a polypeptide having the deduced amino acid sequence translated from the polynucleotide sequence in SEQ ID NO: 1 and variants, fragments, homologues, analogues and derivatives thereof; (b) a polypeptide of SEQ ID NO: 2 and variants, fragments, homologues, analogues and derivatives thereof.

SEQUENCE LISTING

<110> Pfizer Ltd. (EP(GB) only)
      Pfizer Inc. (US, JP, EP except GB)

<120> Novel polypeptide

<130> PCS10960A_BXP

<150> GB 0030855.1
<151> 2000-12-18

<150> GB 0101222.8
<151> 2001-01-17

<160> 7

<170> FastSEQ for Windows Version 4.0

<210> 1
<211> 1082
<212> DNA
<213> homo sapiens

<400> 1
atgctgtcca ttttgcttcc ttccagggga agcagaagcg ggagccgtcg tggagctctg 60
ctcctggagg gagcctcccg ggacatggag aaggtggaca tgaatacatc acaggaacaa 120
ggtctctgcc agttctcaga gaagtacaag caagtctacc tctccctggc ctacagtatc 180
atctttatcc tagggctgcc actaaatggc actgtcttgt ggcactcctg gggccaaacc 240
aagcgctgga gctgtgccac cacctatctg gtgaacctga tggtggccga cctgctttat 300
gtgctattgc ccttcctcat catcacctac tcactagatg acaggtggcc cttcggggag 360
ctgctctgca agctggtgca cttcctgttc tatatcaacc tttacggcag catcctgctg 420
ctgacctgca tctctgtgca ccagttccta ggtgtgtgcc acccactgtg ttcgctgccc 480
taccggaccc gcaggcatgc ctggctgggc accagcacca cctgggccct ggtggtcctc 540
cagctgctgc ccacactggc cttctcccac acggactaca tcaatggcca gatgatctgg 600
tatgacatga ccagccaaga gaattttgat cggctttttg cctacggcat agttctgaca 660
ttgtctggct ttctttccct ccttggtcat tttggtgtgc tattcactga tggtcaggag 720
cctgatcaag ccagaggaga acctcatgag gacaggcaac acagcccgag ccaggtccat 780
ccggaccatc ctactggtgt gtggcctctt caccctctgt tttgtgccct tccatatcac 840
tcgctccttc tacctcacca tctgctttct gctttctcag gactgccagc tcttgatggc 900
agccagtgtg gcctacaaga tatggaggcc tctggtgagt gtgagcagct gcctcaaccc 960
agtcctgtac tttctttcaa ggggggcaaa aatagagtca ggctcctcca gaaactgagg 1020
cagaacaagt tgggtgagca tccagctggg aggaagagat gcccagggtt gaacagatct 1080
gg                                                                1082

<210> 2
<211> 360
<212> PRT
<213> homo sapiens

<400> 2
Met Leu Ser Ile Leu Leu Pro Ser Arg Gly Ser Arg Ser Gly Ser Arg
 1               5                  10                  15
Arg Gly Ala Leu Leu Leu Glu Gly Ala Ser Arg Asp Met Glu Lys Val
            20                  25                  30
Asp Met Asn Thr Ser Gln Glu Gln Gly Leu Cys Gln Phe Ser Glu Lys
        35                  40                  45
Tyr Lys Gln Val Tyr Leu Ser Leu Ala Tyr Ser Ile Ile Phe Ile Leu
    50                  55                  60
Gly Leu Pro Leu Asn Gly Thr Val Leu Trp His Ser Trp Gly Gln Thr
65                  70                  75                  80

Lys Arg Trp Ser Cys Ala Thr Thr Tyr Leu Val Asn Leu Met Val Ala
                85                  90                  95
Asp Leu Leu Tyr Val Leu Leu Pro Phe Leu Ile Ile Thr Tyr Ser Leu
            100                 105                 110
Asp Asp Arg Trp Pro Phe Gly Glu Leu Leu Cys Lys Leu Val His Phe
        115                 120                 125
Leu Phe Tyr Ile Asn Leu Tyr Gly Ser Ile Leu Leu Leu Thr Cys Ile
    130                 135                 140
Ser Val His Gln Phe Leu Gly Val Cys His Pro Leu Cys Ser Leu Pro
145                 150                 155                 160
Tyr Arg Thr Arg Arg His Ala Trp Leu Gly Thr Ser Thr Thr Trp Ala
                165                 170                 175
Leu Val Val Leu Gln Leu Leu Pro Thr Leu Ala Phe Ser His Thr Asp
            180                 185                 190
Tyr Ile Asn Gly Gln Met Ile Trp Tyr Asp Met Thr Ser Gln Glu Asn
        195                 200                 205
Phe Asp Arg Leu Phe Ala Tyr Gly Ile Val Leu Thr Leu Ser Gly Phe
    210                 215                 220
Leu Ser Leu Leu Gly His Phe Gly Val Leu Phe Thr Asp Gly Gln Glu
225                 230                 235                 240
Pro Asp Gln Ala Arg Gly Glu Pro His Glu Asp Arg Gln His Ser Pro
                245                 250                 255
Ser Gln Val His Pro Asp His Pro Thr Gly Val Trp Pro Leu His Pro
            260                 265                 270
Leu Phe Cys Ala Leu Pro Tyr His Ser Leu Leu Leu Pro His His Leu
        275                 280                 285
Leu Ser Ala Phe Ser Gly Leu Pro Ala Leu Asp Gly Ser Gln Cys Gly
    290                 295                 300
Leu Gln Asp Met Glu Ala Ser Gly Glu Cys Glu Gln Leu Pro Gln Pro
305                 310                 315                 320
Ser Pro Val Leu Ser Phe Lys Gly Gly Lys Asn Arg Val Arg Leu Leu
                325                 330                 335
Gln Lys Leu Arg Gln Asn Lys Leu Gly Glu His Pro Ala Gly Arg Lys
            340                 345                 350
Arg Cys Pro Gly Leu Asn Arg Ser
                355                 360

<210> 3
<211> 1020
<212> DNA
<213> Homo sapiens

<400> 3
atgctgtcca ttttgcttcc ttccagggga agcagaagcg ggagccgtcg tggagctctg 60
ctcctggagg gagcctcccg ggacatggag aaggtggaca tgaatacatc acaggaacaa 120
ggtctctgcc agttctcaga gaagtacaag caagtctacc tctccctggc ctacagtatc 180
atctttatcc tagggctgcc actaaatggc actgtcttgt ggcactcctg gggccaaacc 240
aagcgctgga gctgtgccac cacctatctg gtgaacctga tggtggccga cctgctttat 300
gtgctattgc ccttcctcat catcacctac tcactagatg acaggtggcc cttcggggag 360
ctgctctgca agctggtgca cttcctgttc tatatcaacc tttacggcag catcctgctg 420
ctgacctgca tctctgtgca ccagttccta ggtgtgtggc acccactgtg ttcgctgccc 480
taccggaccc gcaggcatgc ctggctgggc accagcacca cctgggccct ggtggtcctc 540
cagctgctgc ccacactggc cttctcccac acggactaca tcaatggcca gatgatctgg 600
tatgacatga ccagccaaga gaattttgat cggctttttg cctacggcat agttctgaca 660
ttgtctggct ttctttcccc ctccttggtc attttggtgt gctattcact gatggtcagg 720
agcctgatca agccagagga gaacctcatg aggacaggca acacagcccg agccaggtcc 780
atccggacca tcctactggt gtgtggcctc ttcaccctct gttttgtgcc cttccatatc 840
actcgctcct tctacctcac catctgcttt ctgctttctc aggactgcca gctcttgatg 900
gcacccagtg tggcctacaa gatatggagg cctctggtga gtgtgagcag ctgcctcaac 960
ccagtcctgt actttctttc aaggggggca aaaatagagt caggctcctc cagaaactga 1020

<210> 4
<211> 339
<212> PRT
<213> Homo sapiens

<400> 4
Met Leu Ser Ile Leu Leu Pro Ser Arg Gly Ser Arg Ser Gly Ser Arg
1               5                   10                  15
Arg Gly Ala Leu Leu Leu Glu Gly Ala Ser Arg Asp Met Glu Lys Val
            20                  25                  30
Asp Met Asn Thr Ser Gln Glu Gln Gly Leu Cys Gln Phe Ser Glu Lys
        35                  40                  45
Tyr Lys Gln Val Tyr Leu Ser Leu Ala Tyr Ser Ile Ile Phe Ile Leu
    50                  55                  60
Gly Leu Pro Leu Asn Gly Thr Val Leu Trp His Ser Trp Gly Gln Thr
65                  70                  75                  80
Lys Arg Trp Ser Cys Ala Thr Thr Tyr Leu Val Asn Leu Met Val Ala
                85                  90                  95
Asp Leu Leu Tyr Val Leu Leu Pro Phe Leu Ile Ile Thr Tyr Ser Leu
            100                 105                 110
Asp Asp Arg Trp Pro Phe Gly Glu Leu Leu Cys Lys Leu Val His Phe
        115                 120                 125
Leu Phe Tyr Ile Asn Leu Tyr Gly Ser Ile Leu Leu Leu Thr Cys Ile
    130                 135                 140
Ser Val His Gln Phe Leu Gly Val Trp His Pro Leu Cys Ser Leu Pro
145                 150                 155                 160
Tyr Arg Thr Arg Arg His Ala Trp Leu Gly Thr Ser Thr Thr Trp Ala
                165                 170                 175
Leu Val Val Leu Gln Leu Leu Pro Thr Leu Ala Phe Ser His Thr Asp
            180                 185                 190
Tyr Ile Asn Gly Gln Met Ile Trp Tyr Asp Met Thr Ser Gln Glu Asn
        195                 200                 205
Phe Asp Arg Leu Phe Ala Tyr Gly Ile Val Leu Thr Leu Ser Gly Phe
    210                 215                 220
Leu Ser Pro Ser Leu Val Ile Leu Val Cys Tyr Ser Leu Met Val Arg
225                 230                 235                 240
Ser Leu Ile Lys Pro Glu Glu Asn Leu Met Arg Thr Gly Asn Thr Ala
                245                 250                 255
Arg Ala Arg Ser Ile Arg Thr Ile Leu Leu Val Cys Gly Leu Phe Thr
            260                 265                 270
Leu Cys Phe Val Pro Phe His Ile Thr Arg Ser Phe Tyr Leu Thr Ile
        275                 280                 285
Cys Phe Leu Leu Ser Gln Asp Cys Gln Leu Leu Met Ala Pro Ser Val
    290                 295                 300
Ala Tyr Lys Ile Trp Arg Pro Leu Val Ser Val Ser Ser Cys Leu Asn
305                 310                 315                 320
Pro Val Leu Tyr Phe Leu Ser Arg Gly Ala Lys Ile Glu Ser Gly Ser
                325                 330                 335
Ser Arg Asn

<210> 5
<211> 27
<212> DNA
<213> Homo sapiens

<400> 5
   accatgctgt ccattttgct tccttcc                                          27

<210> 6
<211> 24
<212> DNA
<213> Homo sapiens

<400> 6
tcaccagatc tgttcaaccc tggg 24

<210> 7
<211> 24
<212> DNA
<213> Homo sapiens

<400> 7
tcagtttctg gaggagcctg actc 24

## Description

### Technical field

The present invention relates to a novel polynucleotide sequence which encodes a novel polypeptide belonging to the class of proteins known as G-protein coupled receptors (GPCRs). The present invention also relates, inter alia, to processes for producing the polypeptide and its uses.

### Background of the invention

Cells and tissues respond to a wide variety of extracellular signalling molecules through the interaction of these molecules with specific cell-surface receptors. One such class of receptors are known as G-protein coupled receptors (GPCRs) and these are characterised by containing a series of 7 hydrophobic transmembrane segments. Upon binding an extracellular ligand to its receptor, intracellular signals are initiated via interactions with heterotrimeric G proteins which in turn can lead to a number of different intracellular events depending upon which receptor has been activated. For example some GPCRs influence adenyl cyclase activity whereas others act via phospholipase C.

Members of the GPCR superfamily respond to a wide variety of ligands including small molecule amines (such as serotonin, dopamine, acetylcholine), lipid-derived mediators (such as LpA), amino acid derivatives (such as glutamate) and neurotransmitter peptides and hormones (such as neurokinin, galanin, glucagon, gastrin). Although GPCRs are activated by a broad range of ligands, it should be noted that individual GPCRs have a small and very specific repertoire of ligands. Based upon an analysis of the primary structure of a novel GPCR, it is now possible to classify them into specific sub-families, thereby narrowing the range of potential ligands.

In many cases, the endogenous ligands of GPCRs are relatively small, enabling them to be mimicked or blocked by synthetic analogues. For example drugs such as prazosin, doxazosin, cimetidine, ranitidine are all effective antagonists of their respective target GPCRs.

Thus, as the activation or inhibition of GPCRs can have therapeutic consequences, there is a continued need to provide new GPCRs and their associated agonists and antagonists.

There are several diverse families of receptors which respond to purines and pyrimidines. Examples of members of the GPCR family of such receptors are the adenosine receptors designated A1, A2a, A2b, and A3; and several of the P2Y receptors which are stimulated by UDP, UTP, ADP, and ATP.

### Summary of the invention

According to one aspect of the present invention, there is provided an isolated polynucleotide comprising:
(a) a polynucleotide encoding the polypeptide as set forth in SEQ ID NO 2;
(b) a polynucleotide encoding the polypeptide as set forth in SEQ ID NO 4;
(c) a polynucleotide comprising a nucleotide sequence of SEQ ID NO 1;
(d) a polynucleotide comprising a nucleotide sequence of SEQ ID NO 3;
(e) a polynucleotide comprising a nucleotide sequence that has at least 70-75% identity to the polynucleotide of any one of (a) to (d);
(f) a polynucleotide comprising a nucleotide sequence which is capable of hybridising to the polynucleotide of any one of (a) to (e); or
(g) a polynucleotide fragment of the polynucleotide of any one of (a) to (f).

Preferably, the polynucleotide comprises a nucleotide sequence that has at least 75-80% identity to the polynucleotide of any one of (a) to (d) above. More preferably, the polynucleotide comprises a nucleotide sequence that has at least 80-85% identity to the polynucleotide of any one of (a) to (d) above. Even more preferably, the polynucleotide comprises a nucleotide sequence that has at least 85-90% identity to the polynucleotide of any one of (a) to (d) above. Yet more preferably, the polynucleotide comprises a nucleotide sequence that has at least 90-95% identity to the polynucleotide of any one of (a) to (d) above. Most preferably, the polynucleotide comprises a nucleotide sequence that has greater than 95% identity to the polynucleotide of any one of (a) to (d) above.

The polynucleotide described above preferably encodes a G-protein coupled receptor (GPCR).

The present invention also provides a polynucleotide probe or primer comprising at least 15 contiguous nucleotides of the polynucleotide described above. The invention also provides antisense oligonucleotides which hybridise to the polynucleotide of SEQ ID NO 1 or SEQ ID NO: 3 and allelic variants thereof and can be used to modify PFI-020 expression. The invention also includes ribozymes which contain portions of sequence capable of hybridising to the polynucleotide of SEQ ID NO 1 or SEQ ID NO: 3.

The present invention yet further provides a vector comprising the polynucleotide described above.

According to a further aspect of the present invention, there is provided a host cell transformed or transfected with the vector described above. Preferably, the host cell is a mammalian, bacterial or yeast cell.

According to yet a further aspect of the present invention, there is provided a process for producing a polypeptide or fragment thereof comprising culturing said host cell under conditions sufficient for the expression of said polypeptide or fragment. Preferably, said polypeptide or fragment is expressed at the surface of said cell. The process preferably further includes recovering the polypeptide or fragment from the culture.

There is also provided by the present invention a process for producing cells capable of expressing a polypeptide or fragment thereof comprising transforming or transfecting cells with the vector described above.

According to a further embodiment of the present invention, there are provided cells produced by the process described above. There is also provided a membrane preparation of said cells.

According to another aspect of the present invention, there is provided a polypeptide comprising:
(a) a polypeptide having the deduced amino acid sequence translated from the polynucleotide sequence in SEQ ID NO 1 and variants, fragments, homologues, analogues and derivatives thereof;
(b) a polypeptide having the deduced amino acid sequence translated from the polynucleotide sequence in SEQ ID NO 3 and variants, fragments, homologues, analogues and derivatives thereof;
(c) a polypeptide of SEQ ID NO 2 and variants, fragments, homologues, analogues and derivatives thereof;
(d) a polypeptide of SEQ ID NO 4 and variants, fragments, homologues, analogues and derivatives thereof; or

There is also provided by the present invention an antibody against the polypeptide described above.

The present invention yet further provides a compound which activates the polypeptide described above (an agonist), preferably a nucleotide or nucleotide derivative, even more preferably 2-chloroadenosine triphosphate tetrasodium (2-chloro-ATP), 2-methylthioadenosine diphosphate trisodium (2-methyl-thio-ADP) 2-methylthioadenosine triphosphate tetrasodium (2-methyl-thio-ATP). The present invention also provides a compound which inhibits activation of the polypeptide described above (an antagonist).

According to another aspect of the invention, there is provided a method for identifying a compound which binds to the polypeptide described above, comprising:
(a) contacting (i) a detectable compound A, preferably a labelled derivative of a purinoceptor ligand known to bind to the polypeptide of the invention, more preferably a nucleotide derivative, even more preferably 2-chloro-ATP, 2-methyl-thio-ATP, or 2-methyl-thio-ADP, and (ii) a test compound (or mixture of test compounds), with cells expressing the polypeptide of the invention or a membrane preparation of such cells;
(b) contacting the same amount of detectable compound A with the same amount of cells expressing the polypeptide of the invention or a membrane preparation of such cells as in step (a) under the same conditions as in step (a) but in the absence of test compound;
(c) comparing the amount of compound A bound in steps (a) and (b) thereby identifying a test compound (or mixture of test compounds) that competes with or blocks the binding of compound A to the polypeptide of the invention.

According to another aspect of the present invention, there is provided a method for identifying a compound which binds to and activates the polypeptide described above comprising:
(a) contacting a compound with cells expressing on the surface thereof the polypeptide or a membrane preparation of said cells, said polypeptide being associated with a second component capable of providing a detectable signal in response to the binding of a compound to said polypeptide; said contacting being under conditions sufficient to permit binding of compounds to the polypeptide; and
(b) identifying a compound capable of polypeptide binding by detecting the signal produced by said second component.

According to another aspect of the present invention, there is provided a method for identifying a compound which binds to and inhibits activation of the polypeptide described above comprising:
(a) contacting (i) a detectable first component known to bind to and activate the polypeptide and (ii) a compound with cells expressing on the surface thereof the polypeptide or a membrane preparation of said cells, said polypeptide being associated with a second component capable of providing a detectable signal in response to the binding of a compound to said polypeptide; said contacting being under conditions sufficient to permit binding of compounds to the polypeptide; and
(b) determining whether the first component binds to the polypeptide by detecting the absence or otherwise of a signal generated from the interaction of the first component with the polypeptide.

As GPCRs are involved in signal transduction, agonists or antagonists of the polypeptide of the present invention can find use in interfering in the signal transduction process. Consequently, the present invention provides a compound which activates the polypeptide described above (an agonist) or which inhibits activation of the polypeptide described above (an antagonist) for use as a pharmaceutical. Such compounds, which can act as agonists or antagonists of the polypeptide, can therefore find use in the therapeutic areas which concern aspects of signal transduction. Therapeutically useful areas include, but are not limited to, neurological disease, psychotherapeutics, urogenital disease, reproduction and sexual medicine, inflammation, cancer, tissue repair, dermatology, skin pigmentation, photoageing, frailty, osteoporosis, metabolic disease, cardiovascular disease, gastrointestinal disease, antiinfection, allergy and respiratory disease, sensory organ disorders, sleep disorders and hairloss. Preferably, the therapeutically useful areas are mood disorders, depression and arousal, even more preferably they are eating disorders and sleep disorders.

Accordingly, there is also provided the use of the above compound (agonist) in the manufacture of a medicament in the treatment of a patient having need to activate a receptor.

There is also provided the use of the above compound (antagonist) in the manufacture of a medicament in the treatment of a patient having need to inhibit a receptor.

According to yet a further aspect of the invention, there is provided a method for the treatment of a patient having need to activate a receptor comprising administering to the patient a therapeutically effective amount of the above-described compound (agonist). Preferably, said compound (agonist) is a polypeptide and a therapeutically effective amount of the compound is administered by providing to the patient DNA encoding said compound and expressing said compound *in vivo.*

According to yet a further aspect of the invention, there is also provided a method for the treatment of a patient having need to inhibit a receptor comprising administering to the patient a therapeutically effective amount of the above-described compound (antagonist). Preferably, said compound (antagonist) is a polypeptide and a therapeutically effective amount of the compound is administered by providing to the patient DNA encoding said compound and expressing said compound *in vivo.*

There is also provided by the present invention a method for the treatment of a patient having need to activate or inhibit a receptor, comprising administering to the patient a therapeutically effective amount of the antibody described above.

Yet further provided by the present invention is use of the antibody described above in the manufacture of a medicament for the treatment of a patient having need to activate or inhibit a receptor.

According to a further aspect of the present invention, there is provided a method of treatment of a patient having need to upregulate a receptor, comprising administering to the patient a therapeutically effective amount of the polypeptide of the present invention. Preferably, said therapeutically effective amount of the polypeptide is administered by providing to the patient DNA encoding said polypeptide and expressing said polypeptide *in vivo.*

There is also provided by the present invention, use of the polypeptide in the manufacture of a medicament for the treatment of a patient having need to upregulate a receptor.

According to yet a further aspect of the present invention, there are provided cells or an animal genetically engineered to overexpress, underexpress or to exhibit targeted deletion of the polypeptide of the present invention.

### Detailed description of the invention

The polynucleotide which encodes the GPCR of the present invention was identified electronically and analysed using various bioinformatic tools. The GPCR encoded by the sequences described herein has been termed PFI-020.

The term "nucleotide sequence" as used herein refers to an oligonucleotide sequence or polynucleotide sequence, and variants, homologues, fragments and derivatives thereof (such as portions thereof). The nucleotide sequence may be DNA or RNA of genomic or synthetic or recombinant origin which may be double-stranded or single-stranded whether representing the sense or antisense strand.

Preferably, the term "nucleotide sequence" means DNA. More preferably, the term "nucleotide sequence" means DNA prepared by use of recombinant DNA techniques (i.e. recombinant DNA).

As used herein "amino acid sequence" refers to peptide or protein sequences or portions thereof.

The present invention does not cover the native PFI-020 according to the present invention when it is in its natural environment and when it has been expressed by its native nucleotide coding sequence which is also in its natural environment and when that nucleotide sequence is under the control of its native promoter which is also in its natural environment. The invention also does not cover the native PFI-020 nucleotide coding sequence which is in its natural environment and under control of its native promoter which is also in its natural environment.

As used herein "naturally occurring" refers to a PFI-020 with an amino acid sequence found in nature.

As used herein "biologically active" refers to a PFI-020 having structural, regulatory or biochemical functions of the naturally occurring PFI-020.

As used herein, "immunological activity" is defined as the capability of the natural, recombinant or synthetic PFI-020 or any oligopeptide thereof, to induce a specific immune response in appropriate animals or cells and to bind with specific antibodies.

The term "antibody" includes polyclonal antibodies, monoclonal antibodies, antibody fragments produced by proteolytic digestion of whole antibody molecules, such as Fab or F(ab')₂ fragments, as well as antibody fragments selected from expression libraries of Fab or single-chain Fv fragments. As the skilled person will be well aware, antibodies can be generated in animals such as mice, rats, rabbits, goats, sheep, etc, by immunising the animal with the polypeptide or oligopeptides selected from the sequence of the polypeptide. If such oligopeptides are used, they are often coupled to carrier proteins ― all methods the skilled person will be familiar with.

Monoclonal antibodies may be prepared using any technique which provides for the production of antibody molecules by continuous cell lines in culture. These include, but are not limited to, the hybridoma technique originally described by Koehler and Milstein (1975, Nature 256, 495-497), the human B-cell hybridoma technique (Kosbor et al. (1983) Immunol Today 4, 72; Cote et al. (1983) Proc. Natl. Acad. Sci. (USA) 80, 2026-2030) and the EBV-hybridoma technique (Cole et al. (1985) Monoclonal Antibodies and Cancer Therapy, Alan R Liss Inc, pp. 77-96). In addition, techniques developed for the production of "chimeric antibodies", the splicing of mouse antibody genes to human antibody genes to obtain a molecule with appropriate antigen specificity and biological activity can be used (Morrison *et al.* (1984) Proc. Natl. Acad. Sci. (USA) 81, 6851-6855; Neuberger et al. (1984) Nature 312, 604-608; Takeda et al. (1985) Nature 314, 452-454). Alternatively, techniques described for the production of single chain antibodies (US-A-4946779) can be adapted to produce polypeptide-specific single chain antibodies.

Antibodies may also be produced by inducing *in vivo* production in the lymphocyte population or by screening recombinant immunoglobulin libraries or panels of highly specific binding reagents as disclosed in Orlandi *et al.* (1989, Proc. Natl. Acad. Sci. (USA) Vol 86 p 3833-3837), and Winter G & Milstein C (1991; Nature 349 p293-299).

The term "derivative" as used herein includes chemical modification of a PFI-020.

As used herein, the terms "isolated" and "purified" refer to molecules, either nucleic or amino acid sequences, that are removed from their natural environment and isolated or separated from at least one other component with which they are naturally associated. For example, for nucleic acid sequences, the nucleic acid must be separated from at least one of the genes with which it is naturally associated.

There are many methods for purifying proteins known to the skilled person, which can be applied to purification of the PFI-020 protein. Often a convenient method involves engineering the cDNA to introduce a sequence encoding a peptide tag, e.g. a hexa-His tag or a Flag peptide tag, either at the 5' end just after the ATG initiation codon, or at the C-terminus before the stop codon, so that the expressed protein will be tagged and can be purified e.g. on a Ni²⁺ chelating column if a hexa-His tag is used, or using commercially available anti-Flag peptide antibodies, e.g. for immunoprecipitation or affinity chromatography techniques. Expression vectors engineered to contain such tags are commercially available, and such methods are well known to the skilled person.

The invention also encompasses purifying and crystallising the polypeptide, optionally followed by elucidating the three-dimensional structure, preferably by X-ray crystallography. The invention also encompasses deriving a homology model of the three-dimensional structure of the polypeptide of the present invention.

Once the protein is purified, crystals may be obtained with methods similar to those described by Palczewski et al in Science 289, 739-745 (2000), and the structure can then be solved by X-ray crystallography as described in this publication, or other biophysical techniques. Alternatively, or additionally, the three-dimensional structure of the polypeptide of the invention can also be modelled by homology modelling, comprising the steps of aligning the sequence of the polypeptide of the invention with the sequence of a similar polypeptide of known structure, preferably rhodopsin, mapping the sequence differences onto the known structure, thereby deriving a model for the three-dimensional structure of the polypeptide of the invention. The three-dimensional structure, derived either by structure determination or by homology modelling, can then be used for designing compounds that may bind to the polypeptide of the invention, or prediction whether compounds will bind to it.

The terms "variant", "homologue" or "fragment" in relation to the amino acid sequence for the preferred polypeptide of the present invention include any substitution of, variation of, modification of, replacement of, deletion of or addition of one (or more) amino acid from or to the sequence providing the resultant polypeptide has PFI-020 activity. In particular, the term "homologue" covers homology with respect to structure and/or function.

The terms "variant", "homologue" or "fragment" in relation to the nucleotide sequence coding for the preferred polypeptide of the present invention include any substitution of, variation of, modification of, replacement of, deletion of or addition of one (or more) nucleic acid from or to the sequence providing the resultant nucleotide sequence codes for or is capable of coding for a polypeptide having PFI-020 activity. In particular, the term "homologue" covers homology with respect to structure and/or function providing the resultant nucleotide sequence codes for or is capable of coding for a receptor having PFI-020 activity. With respect to sequence homology, preferably there is at least 70-75%, more preferably at least 75-80%, more preferably at least 80-85%, more preferably 85-90%, yet more preferably 90-95%, and most preferably greater than 95% identity to the polynucleotide sequence shown in SEQ ID NO 1 or SEQ ID NO 3.

In particular, "homology" as used herein can be determined by commercially available computer programs that produce an optimal alignment between two sequences and then calculate % homology (i.e. when comparing protein sequences, also scoring for conservative substitutions, such as a change between Lysine and Arginine, for which the software will use standard scoring matrices) and % identity (i.e. only counting identical residues) between the aligned sequences. Typical examples of such computer programs are GAP and BESTFIT, which are part of the GCG suite of programs (Devereux et al (1984) Nucl. Acids Res. 12, 387; Wisconsin Package Version 10, Genetics Computer Group, Madison, Wisconsin), or ClustalW (Thompson, J.D. et al (1994) Nucl. Acids Res. 22, 4673-80) which can also be used for multiple sequence alignments.

As used herein, the terms "variant", "homologue", "fragment" and "derivative" also include allelic variations of the sequences.

The term "variant" also encompasses sequences that are complementary to sequences that are capable of hydridising to the nucleotide sequences presented herein. Preferably, the term "variant" encompasses sequences that are complementary to sequences that are capable of hydridising under conditions of medium to high stringency (e.g. 55-65°C and 0.1xSSC {1xSSC = 0.15 M NaCl, 0.015 Na₃ citrate pH 7.0}) to the nucleotide sequences presented herein.

The present invention also covers nucleotide sequences that can hybridise to the nucleotide sequences of the present invention (including complementary sequences of those presented herein). In a preferred aspect, the present invention covers nucleotide sequences that can hybridise to the nucleotide sequence of the present invention under conditions of medium to high stringency (e.g. 55-65°C and 0.1xSSC {1xSSC = 0.15 M NaCl, 0.015 Na₃ citrate pH 7.0}) to the nucleotide sequences presented herein. Such polynucleotides or oligonucleotides may be used as probes, or for amplifying all or part of the sequence of the invention when used as PCR primer. These sequences may also be used to modulate the expression of PFI-020 through antisense techniques or the use of ribozymes. Antisense nucleic acids, preferably oligonucleotide about 10 to 30 bases long, capable of specifically binding to the PFI-020 mRNA transcript, i.e. complementary to the sequence in SEQ ID NO 1, are introduced into cells by standard techniques (e.g. using liposomes), bind to the target nucleotide sequences in the cells and thereby prevents transcription and/or translation of the target sequence. The antisense oligonucleotides are often made more stable by modifications such as using phosphorothioate or methylphosphonate oligonucleotides.

Antisense sequences can also be incorporated into ribozymes such as hammerhead or hairpin ribozymes. These can also be introduced into cells and are thought to cleave the specific transcripts and thereby prevent their translation. Such ribozymes can be introduced into cells by gene therapy approaches, or by standard techniques, e.g. using viral vectors or liposomes. They may also be modified chemically to increase their stability to nuclease digestion.

Details about antisense and ribozyme technologies can be found in textbooks such as I. Gibson (Ed.) Antisense and Ribozyme Methodology, Chapman&Hall; R. Schlingensiepen (1997) Antisense ― From Technology to Therapy: Lab Manual and Textbook, Blackwell Science Inc.; P.C. Turner (Ed.) (1997) Ribozyme Protocols, Humana Press.

The term "vector" includes expression vectors and transformation vectors. The term "expression vector" means a construct capable of *in vivo* or *in vitro* expression. The term "transformation vector" means a construct capable of being transferred from one species to another.

The term "purinoceptor ligand" refers to a known ligand of the family of purinoceptors such as P2Y1 receptors. Examples of such ligands include 2-chloro-ATP, 2-methyl-thio-ATP or 2-methyl-thio-ADP.

Methods how to obtain transgenic animals can be found in I.J. Jackson & C.M. Abbott (Eds) (2000) Mouse Genetics and Transgenics: A Practical Approach, Oxford University Press, and in M.J. Tymms & I. Kola (Eds) (2001) Gene Knockout Protocols (Methods in Molecular Biology, Vol 158), Humana Press.

Methods for gene therapy approaches are covered in T.F. Kresina (2000) An Introduction to Molecular Medicine and Gene Therapy; John Wiley & Sons, and in T. Friedmann (Ed.) (1998) The Development of Human Gene Therapy (Cold Spring Harbor Monograph Series, 36), Cold Spring Harbor Laboratory.

For human use, the compounds of the invention, and their pharmaceutically acceptable salts, can be administered alone but will generally be administered in admixture with a suitable pharmaceutical excipient, diluent or carrier selected with regard to the intended route of administration and standard pharmaceutical practice.

For example, the compounds, and their pharmaceutically acceptable salts, can be administered orally, buccally or sublingually in the form of tablets, capsules, ovules, elixirs, solutions or suspensions, which may contain flavouring or colouring agents, for immediate-, delayed-, modified-, sustained-, pulsed- or controlled-release applications.

The compounds can also be administered parenterally, for example, intravenously, intraarterially, intraperitoneally, intrathecally, intraventricularly, intraurethrally, intrasternally, intracranially, intramuscularly or subcutaneously, or they may be administered by infusion or needleless injection techniques. For such parenteral administration they are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

### EXAMPLES

The present invention will now be described, by way of example only, with reference to the accompanying figures, wherein:
**Figure 1** shows a flow diagram of the bioinformatics analysis of the sequence of PFI-020.
**Figure 2** shows a ClustalW Alignment of PFI-020 with the P2U purinoceptor 1 (P2U1).
**Figure 3** shows the stimulation of PFI-020 with 2-methyl-thio-ADP.
**Figure 4** shows the stimulation of PFI-020 with UTP.
**Figure 5** shows the stimulation of PFI-020 with 2-chloro-ATP.
**Figure 6** shows the stimulation of PFI-020 with 2-methyl-thio-ATP.
**SEQ ID NO: 1** shows the nucleotide sequence coding for PFI-020.
**SEQ ID NO: 2** shows the corresponding amino acid sequence coding for PFI-020.
**SEQ ID NO: 3** shows the nucleotide sequence coding for PFI-020'.
**SEQ ID NO: 4** shows the corresponding amino acid sequence coding for PFI-020'.
**SEQ ID NOS: 5, 6 and 7** show the PCR primers used in the Examples.

### Example 1: The identification of PFI-020

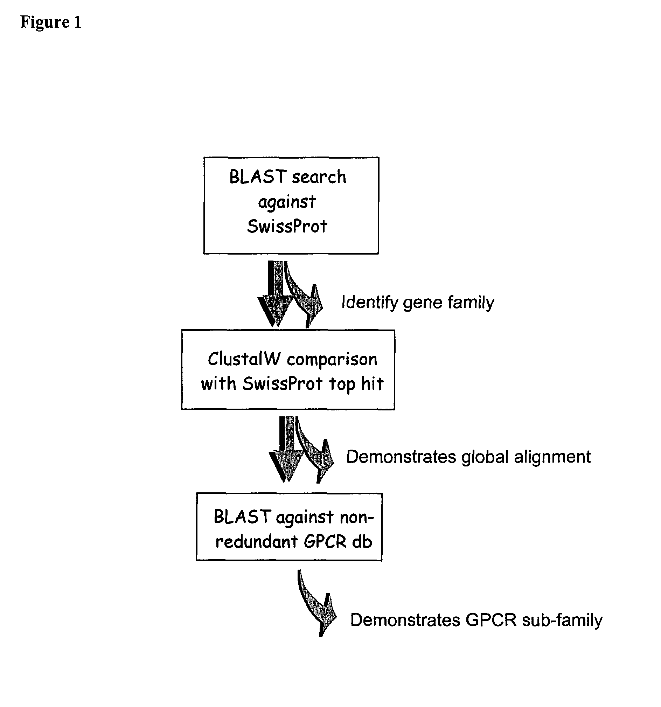

PFI-020 was identified in the Celera nucleotide database by searching the sequences with known members of the G-protein coupled receptor (GPCR) family using the BLAST algorithm. In order to confirm that PFI-020 was a member of the GPCR family, a number of bioinformatics approaches were performed, as shown in Figure 1.

### (a) BLAST Search against Swissprot

PFI-020 was searched against Swissprot using the BLAST algorithm (Basic Local Alignment Search Tool (Altschul SF (1993) J.Mol. Evol. 36:290-300; Altschul, SF et al (1990) J. Mol. Biol. 215:403-410) to identify the closest protein match. In this case the top hit was to SwissProt accession number P41231, P2U purinoceptor 1 (P2U1).

These results indicate that PFI-020 is a member of the GPCR family.

### (b) ClustalW Alignment of PFI-020 with the P2U purinoceptor 1 (P2U1).

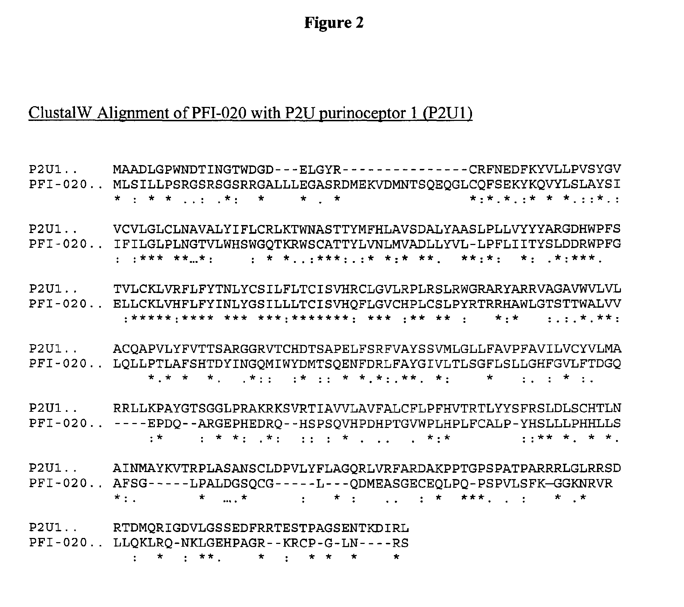

These results are shown in Figure 2. A star (*) underneath the sequence comparison indicates identical residues in both sequences, a colon (:) indicates a conservative difference between the two sequences (e.g. an Arginine residue in one sequence, with the second sequence having a Lysine in the corresponding position); a point (.) underneath indicates that both sequences have similar amino acids in this position (e.g. an Alanine in one, a Valine in the second sequence). The assignment of these symbols is performed by the software according to scoring matrices such as Blosum62, well known to the skilled person.

### (c) BLAST search against a non-redundant human GPCR database

PFI-020 was searched against a non-redundant human GPCR database comprising mainly sequences from Genbank and the Derwent Geneseq databases in order to identify the class of potential agonists for this receptor. The top ten hits are shown below:

| | | e value |
|---|---|---|
| P2U purinoceptor 1 (P2U1) [L:377] | 235 | 5e-63 |
| Uridine nucleotide receptor (UNR) [L:... | 228 | 8e-61 |
| P2Y purinoceptor 6 (P2Y6) [L:328] | 204 | 1e-53 |
| P2Y purinoceptor 1 (P2Y1) [L:373] | 179 | 5e-46 |
| Purinoceptor homologue 6575963CD1 (incyte... | 150 | 3e-37 |
| G protein-coupled receptor GPR17 [L:339] | 147 | 2e-36 |
| P2Y purinoceptor 5 (P2Y5) [L:344] | 139 | 3e-34 |
| P2Y purinoceptor 9 (P2Y9) [L:370] | 139 | 6e-34 |
| Proteinase activated receptor 3 (PAR-3... | 130 | 2e-31 |
| Cysteinyl leukotriene receptor (CYSLT1) ... | 125 | 1e-29 |
| (e value = statistical likelihood of the hit occurring by chance) | | |

These results demonstrate that PFI-020 is most similar to purinergic receptors, and they suggest that PFI-020 encodes a novel GPCR whose ligand is likely to be a nucleotide or nucleotide derivative.

It will be appreciated that the foregoing is provided by way of example only and modification of detail may be made without departing from the scope of the invention.

### Example 2: Isolation of PFI-020

Utilising PFI-020 gene specific primers (PFI-020 forward and PFI-020 reverse; SEQ ID NOs: 5 and 6, respectively) these were employed in a PCR to amplify the PFI-020 coding region from human genomic DNA (Boehringer Mannheim), where the conditions were as follows:-

### PCR mix:

| | |
|---|---|
| PFI-020 primers | 1 µl (10 µM stock) |
| Human genomic DNA | 2 µl (400ng) |
| dNTPs (concentration as per kit) | 1 µl |
| platinum Taq high fidelity Polymerase (LTI, Inc.) | 0.5 µl |
| 10x amplification Buffer (from PCR kit) | 5 µl |
| MgSO₄ | 1.5 µl |
| dH₂O | 39 µl |

### PCR primers:

Forward Primer (= PFI-020 forward):

5'- ACC ATG CTG TCC ATT TTG CTT CCT TCC-3' (SEQ ID NO: 5)

Reverse Primer (= PFI-020 reverse):

5’- TCA CCA GAT CTG TTC AAC CCT GGG-3’ (SEQ ID NO: 6)

### PCR cycle:

(1) 94°C 2 mins
(2) 94°C 30 seconds
(3) 54°C 30 seconds
(4) 68°C 2 mins
   Steps (2) through to (4) were repeated for a further 27 cycles.
(5) 68°C 15 mins
(6) 4°C soak.

The PFI-020 PCR product was TOPO cloned (Invitrogen TOPO cloning methodology) into the vector pcDNA4.1/His-Max-TOPO (Invitrogen), according to the manufacturer's instructions. The resulting insert was subsequently sequence-verified on both strands using ABI DNA sequencing methodology as per the manufacturer's protocol.

### Example 3: Isolation of PFI-020'

PFI-020' was isolated as described in Example 2, except that the reverse primer used had the following sequence:

5'- TCA GTT TCT GGA GGA GCC TGA CTC-3' (SEQ ID NO: 7)

### Example 4: Tissue distribution of PFI-020

Electronic northern identifies an EST in a brain cDNA library.

### Example 5: Functional cell-based assays for agonist activation of PFI-020

Fluorescence Imaging Plate Reader (FLIPR®) technology was employed as a means to detect activation of PFI-020 by agonists in a cell-based assay.

5 x 10⁶ Human Embryonic Kidney (HEK) 293 cells expressing the mouse Gα15 gene (from here on called '293 cells'), were transiently transfected with 7.5 µg of PFI-020 (contained within the pcDNA4HIS-max-TOPO (Invitrogen) plasmid) vector, or vector alone, using Lipofectamine Plus® reagent (Gibco BRL) as per the manufacturer's protocol. The plasmid pcDNA4HIS-max-TOPO was used as it contains elements that up-regulate the level of gene transcription over standard pcDNA3.1 vectors. 24 hrs post-transfection, the cells were detached from the flask using Trypsin/EDTA solution (LTI) and seeded into a black sided, poly-D-lysine-treated, 96-well plate (Becton Dickinson) at 5 x 10⁴ cells/well density. The plates were left overnight to allow the cells to adhere to the bottom of the wells. The medium was removed from the cells and replaced with 100 µl warm (37°C) dye loading solution (50 µg Fluo3 (Molecular Probes) in 20 µl DMSO + 20% pluronic acid in DMSO, added to 11 ml Dulbecco's Modified Eagles Medium containing 1x Probenecid (100x Probenecid - 0.71 g Probenecid was dissolved in 5 ml 1M NaOH and 5 ml Dulbeccos' Phosphate Buffered Saline (PBS), per plate; Probenecid (Molecular Probes) inhibits activity of the anion transport protein, thus improving dye loading). The plates were then incubated for 1 hr at 37°C. Plates were subsequently washed with 250 µl of wash buffer per well (5 ml 100x Probenecid stock + 495 ml PBS, pH 7.4) 4 times. The plates were returned to the 37°C/5%CO₂ incubator for 30 mins prior to processing within the FLIPR® instrument. The FLIPR® processing involved reading the fluorescence for all samples for 2 minutes; during this time the fluorescence baseline was determined for the first 10 seconds. The desired amount of compound was then automatically transferred to the wells and the fluorescence was continuously monitored for the remainder of the time. All compounds were diluted in wash buffer

### Analysis of PFI-020 activation by various purinoceptor agonist compounds in a FLIPR® cell-based assay

Using the methodology as described in detail above, purinoceptor agonist compounds were identified as being able to functionally activate PFI-020.

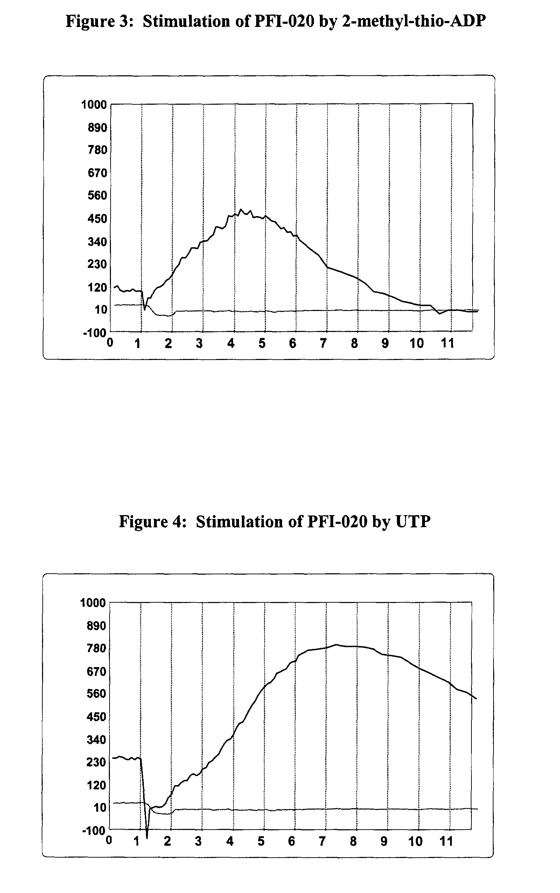

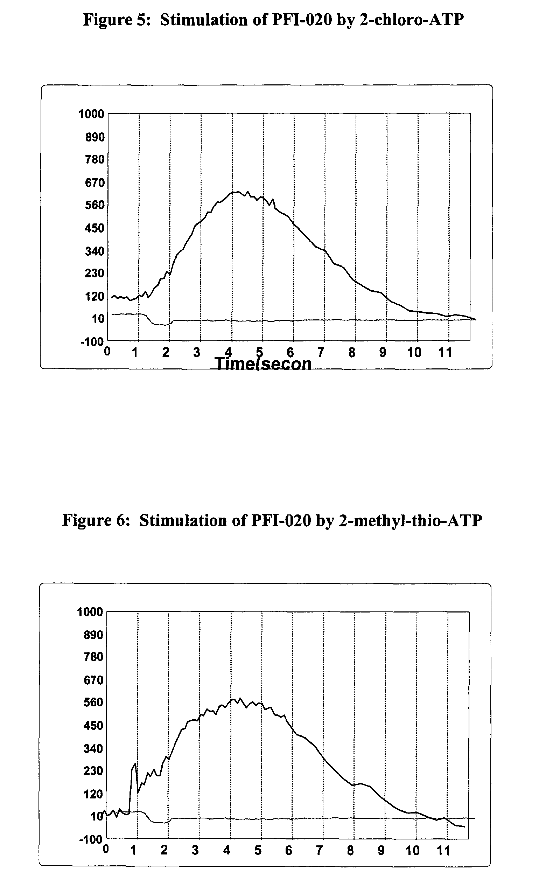

Figures 3, 4, 5 and 6 depict the action of various purinoceptor compounds at a concentration of 10 µM on PFI-020-transfected 293 cells, showing fluorescence intensity versus time (in seconds). Vector-only transfected 293 cells gave no measurable response to these compounds as shown by the grey line in Figures 3-6. The results indicate that PFI-020 is activated, by 2-methylthioadenosine diphosphate trisodium (2-methyl-thio-ADP, black line in graph in Figure 3), Uridine triphosphate (UTP; black line in graph in Figure 4); 2-chloroadenosine triphosphate tetrasodium (2-chloro-ATP, black line in graph in Figure 5); 2-methylthioadenosine triphosphate tetrasodium (2-methyl-thio-ATP, black line in graph in Figure 6). All compounds were purchased from Sigma.

### Example 6: Engineering of stable cell lines expressing high levels of PFI-020

A suitable host cell line, e.g. HEK293 cells or CHO cells (engineered to express a desired G protein such as Gα15), is transfected as described in Example 5, using Lipofectamine or electroporation, with a suitable mammalian cell expression vector containing the cDNA (preferably without any 5' or 3' untranslated regions) encoding PFI-020, and containing a selectable marker, e.g. a neomycin resistance gene. Following transfection, selection pressure is applied, e.g. by adding 400-800 µg/ml G418 to the growth medium and thereby killing all cells which have not taken up the vector which contains the neomycin resistance gene. After about 3-4 weeks of selection, individual clones are picked and expanded for further analysis. The individual clones can be analysed e.g. by Northern blot, using a labelled probe designed from the PFI-020 cDNA sequence.

### Example 7: Ligand binding assays

Cells expressing PFI-020, either 24-72 hours after transient transfection as described in Example 5, or engineered as described in Example 6, are harvested by scraping, resuspended in 20 ml of ice-cold assay buffer (50 mM Tris-HCl pH 7.4), homogenised, and the resulting suspension is centrifuged at 20,000g, 4°C for 30 minutes. The supernatant is decanted, the pellet resuspended in 3 ml of assay buffer and re-homogenised (50 mM Tris-HCl pH7.4). The protein concentration is determined via Bradford's assay (Biorad), according to the manufacturer's recommendations.

Aliquots of this membrane preparation containing 200 µg protein are then incubated with various potential ligands, such as nucleotides, nucleotide analogues, radiolabeled to high specific activity, for about 2 hrs at room temperature or at 30°C (the optimal conditions, ion concentrations, incubation time and temperature need to be determined for each ligand). To terminate incubations, samples are rapidly filtered using the Brandell cell harvester onto Wallac Filtermats (Perkin Elmer) (which have been previously soaked (for 1h) in a 0.3% (v/v) solution of PEI (polyethylenimine; Sigma) in assay buffer to reduce Filtermat binding). Immediately, the Filtermat/wells are washed four times in rapid succession with 2 ml of assay buffer per well. Filtermats are dried using a microwave oven, and Meltilex scintillant (Perkin Elmer) is melted onto the Filtermats using the Wallac Meltilex heat sealer. The bound radioactivity on the Filtermats is determined using the Wallac betaplate scintillation counter.

The specific binding is defined as the difference between total radioactivity bound minus the radioactivity measured in the presence of an excess of unlabelled ligand. Mock-transfected cells are also measured to assess whether the host cells express receptors for the ligands used endogenously.

### Example 7 : (β-lactamase assay

A CHO cell line engineered to stably contain cyclic AMP response elements (CRE) functionally linked to the coding region of reporter gene (β-lactamase as well as the nuclear factor of activated T-cell promoter NF-AT (Flanagan et al (1991) Nature 352, 803-807) linked to the coding region of reporter gene β-lactamase (CHO-CRE-NFAT-BLA) is transfected stably as described in Example 6, with a plasmid containing the cDNA encoding PFI-020 functionally linked to a promoter that drives expression in mammalian cells, e.g. pcDNA3.1, and selected for stable expression of PFI-020.

The CHO-CRE-NFAT-BLA cells expressing PFI-020 are then seeded at 4x 10³ cells per well in 96-well plates, and incubated for 60 hours at 37°C in a CO₂ incubator (5% CO₂). The medium is then removed, and 90 µl starvation medium (DMEM with high glucose, 0.1mM Non-essential amino acids, 1mM sodium pyruvate, 25mM Hepes buffer, without serum or antibiotics) is added to each well, and the cells are incubated overnight. The cells are then stimulated by addition of 10 µl 2-chloro-ATP or 2-methyl-thio-ATP (or 1 µM ionomycin for positive control) prepared in DMEM with 1% dialysed fetal bovine serum per well. Following incubation at 37°C/5% CO₂ for 5 hours, 20µl of 6x dye solution (CCF2 Loading kit from Aurora, Cat # 00 100 012, contains solutions A-D; to prepare 6x dye solution, 36µl solution A (CCF2-AM), 180µl solution B, 2.8ml solution C and 225µl solution D are mixed according to the instructions) are added per well, and the plate is incubated on a rocking platform in the dark at room temperature for 1 hour (rocking at 40 cycles per minute). The fluorescence is then measured in a Cytofluor 4000 (PerSeptive Biosystems), using an excitation wavelength of 405 nm, and measuring emission at wavelengths of 450 nm and 530 nm.

When the ligand stimulates the receptor and the response leads to either a change in cAMP concentration or in calcium concentration in the cells, β-lactamase will be expressed in the cells. The dye is composed of a blue (coumarin) and a green (fluorescein) component which are linked by a β-lactam linker group. When excited at 405 nm, fluorescence energy transfer will occur within the uncleaved molecule, and the emission wavelength will be green (around 530 nm). When the linker is cleaved by β-lactamase, no energy transfer can occur, and blue fluorescence results, measured at 450 nm. Measuring the ratio of blue to green fluorescence will give an indication of receptor stimulation. The ratio is agonist dose dependent, and can be used to rank agonists for the receptor.

## Claims

1. An isolated polynucleotide comprising:
(a) a polynucleotide encoding the polypeptide as set forth in SEQ ID NO 2;
(b) a polynucleotide encoding the polypeptide as set forth in SEQ ID NO 4;
(c) a polynucleotide comprising a nucleotide sequence of SEQ ID NO 1;
(d) a polynucleotide comprising a nucleotide sequence of SEQ ID NO 3;
(e) a polynucleotide comprising a nucleotide sequence that has at least 70-75% identity to the polynucleotide of any one of (a) to (d);
(f) a polynucleotide comprising a nucleotide sequence which is capable of hybridising to the polynucleotide of any one of (a) to (e); or
(g) a polynucleotide fragment of the polynucleotide of any one of (a) to (f).

2. The polynucleotide of claim 1 which encodes a G-protein coupled receptor (GPCR).

3. A polynucleotide probe or primer comprising at least 15 contiguous nucleotides of the polynucleotide of claim 1 or claim 2.

4. A vector comprising the polynucleotide of any one of the preceding claims.

5. A host cell transformed or transfected with the vector of claim 4.

6. The host cell of claim 5 which is a mammalian, bacterial or yeast cell.

7. A process for producing a polypeptide or fragment thereof comprising culturing the host cell of claim 5 or claim 6 under conditions sufficient for the expression of said polypeptide or fragment.

8. The process of claim 7, wherein said polypeptide or fragment is expressed at the surface of said cell.

9. Cells produced by the process of claim 7 or claim 8.

10. A membrane preparation of the cells of claim 9.

11. A polypeptide comprising:
(a) a polypeptide having the deduced amino acid sequence translated from the polynucleotide sequence in SEQ ID NO 1 and variants, fragments, homologues, analogues and derivatives thereof; or
(b) a polypeptide of SEQ ID NO 2 and variants, fragments, homologues, analogues and derivatives thereof; or

12. An antibody against the polypeptide of claim 11.

13. A compound (agonist) which activates the polypeptide of claim 11.

14. A compound (antagonist) which inhibits activation of the polypeptide of claim 11.

15. A method for identifying a compound which binds to the polypeptide of claim 11 comprising:
(a) contacting (I) a detectable compound A known to bind to the polypeptide of claim 11, and (ii) a test compound with cells expressing the polypeptide of claim 11 or a membrane preparation of such cells;
(b) contacting the same amount of detectable compound A with the same amount of cells expressing the polypeptide of claim 11 or a membrane preparation of such cells under the same conditions as in step (a) but in the absence of test compound;
(c) comparing the amount of compound A bound in steps (a) and (b) thereby identifying a test compound that competes with or blocks the binding of compound A to the polypeptide of claim 11.

16. The method of claim 15, whereby compound A is a nucleotide or nucleotide derivative.

17. A method for identifying a compound which binds to and activates the polypeptide of claim 11 comprising:
a) contacting a compound with cells expressing on the surface thereof the polypeptide of claim 11 or a membrane preparation of said cells, said polypeptide being associated with a second component capable of providing a detectable signal in response to the binding of a compound to said polypeptide; said contacting being under conditions sufficient to permit binding of compounds to the polypeptide; and
b) identifying a compound capable of polypeptide binding by detecting the signal produced by said second component.

18. A method for identifying a compound which binds to and inhibits activation of the polypeptide of claim 11 comprising:
(a) contacting (I) a detectable first component known to bind to and activate the polypeptide of claim 11 and (ii) a compound with cells expressing on the surface thereof the polypeptide of claim 11, or a membrane preparation of said cells, said polypeptide being associated with a second component capable of providing a detectable signal in response to the binding of a compound to said polypeptide; said contacting being under conditions sufficient to permit binding of compounds to the polypeptide; and
(b) determining whether the first component binds to the polypeptide by detecting the absence or otherwise of a signal generated from the interaction of the first component with the polypeptide.

19. The compound of claim 13 or claim 14 for use as a pharmaceutical.

20. Use of the compound (agonist) of claim 13 in the manufacture of a medicament in the treatment of a patient having need to activate a receptor.

21. Use of the compound (antagonist) of claim 14 in the manufacture of a medicament in the treatment of a patient having need to inhibit a receptor.

22. Use of the antibody of claim 12 in the manufacture of a medicament for the treatment of a patient having need to activate or inhibit a receptor.

23. Use of the polypeptide of claim 11 in the manufacture of a medicament for the treatment of a patient having need to upregulate a receptor.

24. Cells or animals genetically engineered to overexpress or to underexpress the polypeptide of claim 11.

25. Cells or animals genetically engineered to exhibit targeted deletion of the polypeptide of claim 11.

26. A method of elucidating the three-dimensional structure of the polypeptide of claim 11, comprising the steps of: (a) purifying the polypeptide; (b) crystallising it, and (c) elucidating the structure, in particular by X-ray crystallography.

27. A method of modelling the structure of the polypeptide of claim 11, comprising the steps of: (a) aligning the sequence with a sequence of a protein of known three-dimensional structure, in particular rhodopsin; (b) mapping the detected sequence differences of the polypeptide of claim 11 onto the known structure, (c) deriving a homology model of the polypeptide of claim 11.
